# EUROPEAN PATENT SPECIFICATION

(11) **EP 1 073 749 B1**
(45) Date of publication and mention of the grant of the patent: **11.01.2006**
(21) Application number: 99918767.7
(22) Date of filing: 22.04.1999
(51) Int. Cl.: C12N 15/36, C12N 15/49, A61K 48/00, A61K 39/39

(54) **ENHANCING IMMUNE RESPONSES TO GENETIC IMMUNIZATION BY USING A CHEMOKINE**
ERHÖHUNG DER IMMUNANTWORTEN BEI GENETISCHER IMMUNISIERUNG DURCH VERWENDUNG EINES CHEMOKINS
PROCEDE POUVANT AMELIORER DES REPONSES IMMUNITAIRES A UNE IMMUNISATION GENETIQUE

(30) Priority: 22.04.1998 US 82600 P
(43) Date of publication of application: 07.02.2001
(73) Proprietor: CHIRON CORPORATION, Emeryville, California 94608-2916 (US)
(72) Inventor: PALIARD, Xavier, Emeryville, CA 94622-8097 (US)
(74) Representative: Hallybone, Huw George
(86) International application number: PCT/US1999/008802
(87) International publication number: WO 1999/053960

(56) References cited:
- WO-A-94/28916
- WO-A-96/11279
- WO-A-96/36366
- WO-A-97/19696
- WO-A-98/15285
- WO-A-99/29728
- KHUDYAKOV Y E ET AL: "LINEAR B-CELL EPITOPES OF THE NS3-NS4-NS5 PROTEINS OF THE HEPATITISC VIRUS AS MODELED WITH SYNTHETIC PEPTIDES" VIROLOGY, vol. 206, 1 January 1995 (1995-01-01), pages 666-672, XP000574456 ISSN: 0042-6822
- MOLDOVEANU Z ET AL: "IMMUNE RESPONSES INDUCED BY ADMINISTRATION OF ENCAPSIDATED POLIOVIRUS REPLICONS WHICH EXPRESS HIV-1 GAG AND ENVELOPE PROTEINS" VACCINE, vol. 13, no. 11, 1 August 1995 (1995-08-01), pages 1013-1022, XP000571592 ISSN: 0264-410X
- DILLOO ET AL: "COMBINED CHEMOKINE AND CYTOKINE GENE TRANSFER ENHANCES ANTITUMOR IMMUNITY" NATURE MEDICINE, vol. 2, no. 10, October 1996 (1996-10), pages 1090-1095, XP002119425

## Description

### TECHNICAL AREA OF THE INVENTION

The invention relates to the area of immune responses to genetic immunization. More particularly, the invention relates to enhancing immune responses to DNA immunogens using immune co-stimulatory molecules.

### BACKGROUND OF THE INVENTION

The use of genetic immunization, or immunization with DNA encoding polypeptide immunogens, to prime immune responses is viewed as a promising vaccine strategy. This technology offers potential improvements compared to other types of vaccines, such as subunit proteins complexed with adjuvants or inactivated or attenuated viral preparations. In addition to the practical advantages of simplicity of construction and modification, injection of genetic material encoding for polypeptide immunogens results in synthesis of the immunogens in the host. Thus, these immunogens are presented to the host immune system with native post-translational modifications, structure, and conformation.

In mice, several DNA vaccines have been effective at inducing long-lived antibody and cytotoxic T lymphocyte (CTL) responses and have conferred protective immunity against a number of viruses, bacteria, parasites, and tumors (1-8). Various approaches to enhance immune responses mediated by genetic immunization have been investigated. In addition to variations in dosage, route or boosting regimens, these variations include co-injection of polynucleotides encoding co-stimulatory molecules which improve immunogen presentation to lymphocytes, such as B7-1 or B7-2, or cytokines, such as GM-CSF, IL-2, IL-2, and IL-12, to create an optimal cytokine microenvironment for T cell priming (11-19). However, further enhancement of immune responses to genetic immunization is desirable for immunizing mammals, particularly humans, against immunogens such as virus- and tumor-specific immunogens.

Thus, there is a need in the art for methods of enhancing the immune responses to DNA immunogens.

### SUMMARY OF THE INVENTION

It is an object of the invention to provide a method of enhancing an immune response to a DNA immunogen. This and other objects of the invention are provided by one or more of the embodiments described below.

One embodiment of the invention provides an immunogenic composition. The composition comprises a DNA immunogen and a B lymphocyte chemokine (BLC) or a polynucleotide encoding a BLC.

Another embodiment of the invention provides a method of enhancing an immune response to a DNA immunogen in a mammal. A BLC or a first polynucleotide encoding a BLC and a DNA immunogen are administered to the mammal. An immune response to the DNA immunogen is thereby enhanced.

The present invention thus provides the art with the information that B lymphocyte chemokines can be used to enhance an immune response of a mammal to a DNA immunogen. The invention can be used to, *inter alia,* to immunize or vaccinate a mammal against an infectious disease or a tumor.

### BRIEF DESCRIPTION OF THE DRAWINGS

**Figure 1.** Figure 1 shows the immunization and bleeding schedules for animals immunized with HCV immunogens.
**Figure 2.** Figure 2 shows the immunization and bleeding schedules for animals immunized with granulocyte-macrophage colony-stimulating factor (GM-CSF).
**Figure 3.** Figure 3 shows the immunization and bleeding schedules for animals immunized with HCV immunogens and RANTES.
**Figure 4.** Figure 4 shows the immunization and bleeding schedules for animals immunized with HCV immunogens and macrophage inflammatory protein 1α (MIP-1α).
**Figure 5.** Figure 5 shows the increased anti-HIV gag antibody titer in mice immunized with a plasmid encoding HIV gag and a plasmid encoding the chemokine B lymphocyte chemokine (BLC).

### DETAILED DESCRIPTION OF THE PREFERRED EMBODIMENTS

It is a discovery of the present invention that administration of a B lymphocyte chemokine (BLC) or a polynucleotide encoding a BLC can be used to enhance an immune response in a mammal to a DNA immunogen. This method can be used, *inter alia*, to increase immunological resistance to pathogens, such as viruses and bacteria, and to tumor-associated immunogens.

Chemokines generally function as chemoattractants for cells which they recruit from the blood to sites of infection. Thus, administration of a chemokine, either together with or in addition to a DNA immunogen, effectively recruits various cell populations, including antigen presenting cells and effector cells, to the site of administration or its vicinity. Similarly, administration of a polynucleotide encoding a chemokine can result in local chemokine secretion which induces migration of antigen presenting cells and/or lymphocytes to the site of administration and which enhances immune responses to the DNA immunogen. Local chemokine secretion can also enhance the migration of cells which have taken up the DNA immunogen or polypeptides encoded by the DNA immunogen to the lymph nodes, where priming of specific T cells can occur.

The chemokine which is used in the method of the invention is a B lymphocyte chemokine (BLC). Optimization of the DNA immunogen-chemokine combination can be carried out using routine assays in standard animal models (see Examples 1 and 2).

The immune response which is enhanced can be any response which is influenced by B lymphocyte chemokines, including, but not limited to, antibody production or cytotoxic T lymphocyte (CTL) response resulting from chemoattraction and/or activation of antigen presenting cells, such as dendritic cells, macrophages, and monocytes, chemoattraction and/or activation of neutrophils, including eosinophils, and chemoattraction and/or activation of naive T cells, memory T cells, and pre-T cells to the thymus.

Measurement of enhanced immune responses can be carried out as is known in the art. For example, antibody titer can be measured by assays such as agglutination, immunoprecipitation, or ELISA.

Assays for chemotaxis relating to neutrophils are described in Walz *et al*. (1987), *Biochem. Biophys. Res. Commun. 149:* 755; Yoshimura et al. (1987), *Proc. Natl. Acad Sci. USA 84:* 9233, and Schroder *et al*. (1987), *J. Immunol. 139:* 3474. Chemotaxis of lymphocytes can be assayed as described in Larsen *et al*., *Science 243:* 1464: (1989) and Carr *et al*., *Proc. Natl. Acad Sci. USA 91:* 3652 (1994).

Assays for chemotaxis of tumor-infiltrating lymphocytes are described in Liao *et al*. (1995), *J. Exp. Med. 182*: 1301; for hemopoietic progenitors, in Aiuti *et al*. (1997), *J. Exp. Med 185*: 111; for monocytes, in Valente *et al*. (1988), *Biochem. 27:* 4162; and for natural killer cells, in Loetscher *et al*. (1996), *J. Immunol. 156:* 322, and in Allavena *et al.* (1994), *Eur. J. Immunol. 24:* 3233.

Attraction or activation of eosinophils, dendritic cells, basophils, and neutrophils, can also be measured. Assays for determining eosinophil attraction are described in Dahinden *et al*., *J. Exp. Med. 179*: 751 (1994), Weber *et al*., *J. Immunol. 154*: 4166 (1995), and Noso *et al*., *Biochem. Biophys. Res. Commun. 200*: 1470 (1994). Attraction of dendritic cells can be measured as described, for example, in Sozzani *et al*., *J. Immunol*. 155: 3292 (1995). Assays for attracting basophils are taught in Dahinden *et al*., *J. Exp. Med. 179*: 751 (1994), Alam *et al*., *J. Immunol*. 152: 1298 (1994), and Alam *et al*., *J. Exp. Med. 176*: 781 (1992). Activation ofneutrophils is taught in Maghazaci *et al*., *Eur. J. Immunol. 26:* 315 (1996) and Taub *et al., J. Immunol. 155*: 3877 (1995). Cytotoxic T lymphocyte assays can also be used to measure enhanced immune response to a DNA immunogen (see Example 1, below)..

The DNA immunogen can be any contiguous sequence of deoxyribonucleotides encoding a polypeptide which is capable of eliciting an immune response. For example, polynucleotides encoding immunogenic polypeptides of viruses such as HIV viruses (*e*.*g*, gag, pol, or env), herpes viruses (*i*.*e*., HSV-1, HSV-2), Epstein-Barr virus, varicella-zoster virus, cytomegalovirus, and hepatitis B virus (HBV), hepatitis C virus (HCV), and human papilloma viruses (*i*.*e*., HPV-16, -18, and -31) can serve as a DNA immunogen. DNA which encodes polypeptide immunogens of other infectious agents, such as bacteria, fungi, or yeast, can function as a DNA immunogen in the method of the invention. DNA which encodes polypeptides specifically expressed by a tumor, such as EGFRvIII, Ras, or p185^{HER2}, or polypeptides which are expressed both by a tumor and by the corresponding normal tissue, can also function as a DNA immunogen. If desired, a DNA immunogen can comprise coding sequences for more than one immunogenic polypeptide.

A B lymphocyte chemokine (BLC) and a DNA immunogen can be administered to a mammal, preferably a human, by any means known in the art, including parenteral, intranasal, or intramuscular injection, or coated onto small metal projectiles and injected using a biological ballistic gun ("gene gun"). Alternatively, a B lymphocyte chemokine and a DNA immunogen can be administered successively. The BLC can be administered prior to administration of the DNA immunogen, or the DNA immunogen can be administered prior to the administration of the BLC.

A polynucleotide encoding the B-lymphocyte chemokine can also be administered. Preferably, a polynucleotide encoding the BLC and a polynucleotide comprising the DNA immunogen are co-injected. The polynucleotides can also be administered successively, in any order. For co-administration, a single polynucleotide comprising both BLC-encoding sequences and the DNA immunogen can be administered, or the DNA immunogen and the BLC-encoding polynucleotide can be provided separately and mixed together prior to administration.

The invention also provides immunogenic compositions comprising a DNA immunogen and a B lymphocyte chemokine or a polynucleotide encoding a B lymphocyte chemokine. The composition can optionally coniprise a pharmaceutically acceptable carrier. Pharmaceutically acceptable carriers are well known to those in the art. Such carriers include, but are not limited to, large, slowly metabolized macromolecules, such as proteins, polysaccharides, polylactic acids, polyglycolic acids, polymeric amino acids, amino acid copolymers, and inactive virus particles. Pharmaceutically acceptable salts can also be used in compositions of the invention, for example, mineral salts such as hydrochlorides, hydrobromides, phosphates, or sulfates, as well as salts of organic acids such as acetates, proprionates, malonates, or benzoates. Compositions of the invention can also contain liquids, such as water, saline, glycerol, and ethanol, as well as substances such as wetting agents, emulsifying agents, or pH buffering agents. Liposomes, such as those described in U.S. 5,422,120, WO 95/13796, WO 91/14445, or EP 524,968 B1, can also be used as a carrier for a composition of the invention.

Compositions of the invention can be used as vaccine compositions, for example, to enhance an immune response of a mammal, including a human, to an infectious agent or a tumor. The particular dosages of BLC and DNA immunogen which are sufficient to enhance an immune response to the DNA immunogen will vary according to the mammal to which the BLC and DNA immunogen are being administered. The amounts of each active agent in the examples described below provide general guidance for the range of each component to be utilized by the practitioner upon optimizing the method of the present invention for practice either *in vitro* or *in vivo*. Generally, 0.1, 0.2, 0.3, 0.4, 0.5, 0.75, 1.0, 1.5, 2.0, 2.5, or 5 mg of a BLC protein, a polynucleotide encoding a BLC, or a polynucleotide comprising a DNA immunogen will be administered to a large mammal, such as a baboon or a human.

Such ranges by no means preclude use of a higher or lower amount of a component, as might be warranted in a particular application. For example, the actual dose and schedule may vary depending on whether the compositions are administered in combination with other pharmaceutical compositions or depending on individual differences in pharmacokinetics, drug disposition, and metabolism.

The following are provided for exemplification purposes only and are not intended to limit the scope of the invention described in broad terms above.

### EXAMPLE 1

### Co-administration of HCV immunogens and MIP-1α increases lysis of autologous B cells infected with vaccinia virus encoding HCV polypeptide NS3

*HCV immunogens.* Each plasmid comprises a CMV enhancer/promoter and is Kanamycin-resistant. Plasmids were prepared by an alkaline lysis method from *E. coli* bacteria and purified using Qiagen purification systems. After purification, plasmids were stored at -80 °C, at a concentration of 1 mg/ml.

Plasmid pCMVKmΔNS comprises hepatitis C viral DNA encoding HCV polypeptides ΔNS3, NS4, NS5a, and NS5b (immunogen for animal Group 1). Plasmid NS-GM2 encodes HCV polypeptides ΔNS3, NS4, NS5b, NS5b, and hGM-CSF (immunogen for animal Group 2). Plasmid pCMVLhRantes encodes human RANTES protein. pCMVLhMIP1a encodes MIP-1α.

For the immunization protocols described below, pCMVKmΔNS was premixed with either pCMVLhRantes (immunogen for animal Group 3) or pCMVLhMIP1a (immunogen for animal Group 4). Each plasmid was at a concentration of 1 mg/ml of DNA, for a total of 2 mg/ml of DNA per mixed immunogen.

*Injection of HCV immunogens into baboons.* On the day of injection, one vial (marked with the plasmid name and animal group) per animal was removed from the freezer, thawed at room temperature, and gently mixed. Each immunogen was injected both intramuscularly and intradermally. The total volume injected per animal was 1 ml.

The left and right tibialis anterior muscle was injected with 400 µl of DNA for a total of 800 µl intramuscular injection per baboon, using a 1 ml syringe. The immunogens were injected slowly, over about 10 seconds. After injection, the needle was removed slowly, to reduce leakage.

Each of two separates sites of the upper back was injected with 100 µl of DNA for a total of 200 µl intradermal injection per baboon, using a 0.3 ml U-100 Insulin syringe. The skin at the sites of injection was shaved. At each site, the needle was inserted the needle bevel up into the skin and then rotated 90 degrees so that the bevel pointed to the side. The 100 µl was slowly injected over about 10 seconds. After injection, the needle was slowly rotated so that the bevel was up again, then withdrawn slowly to reduce leakage.

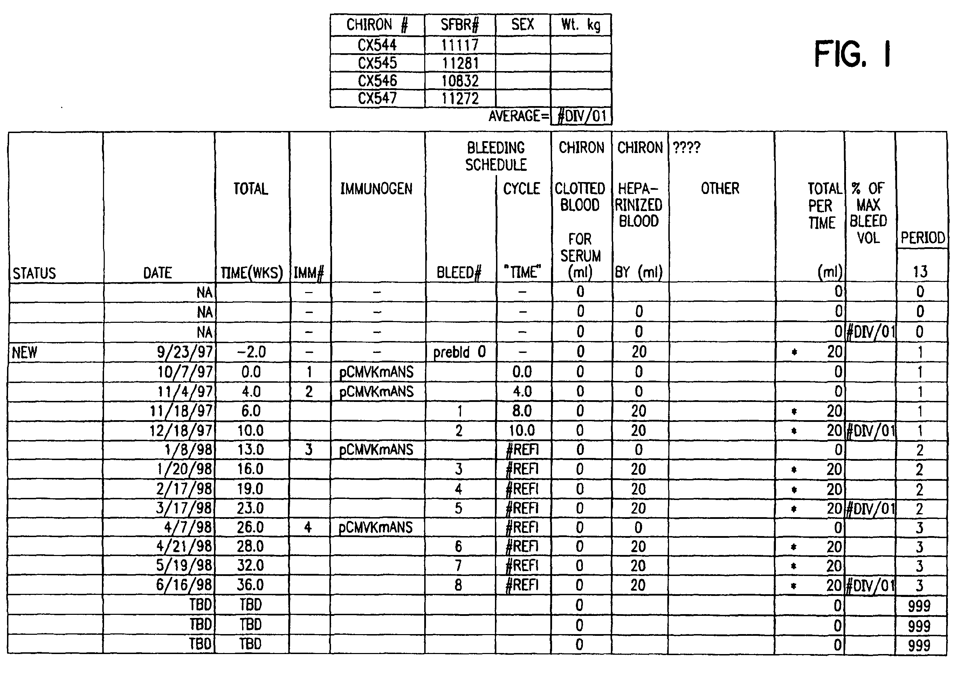

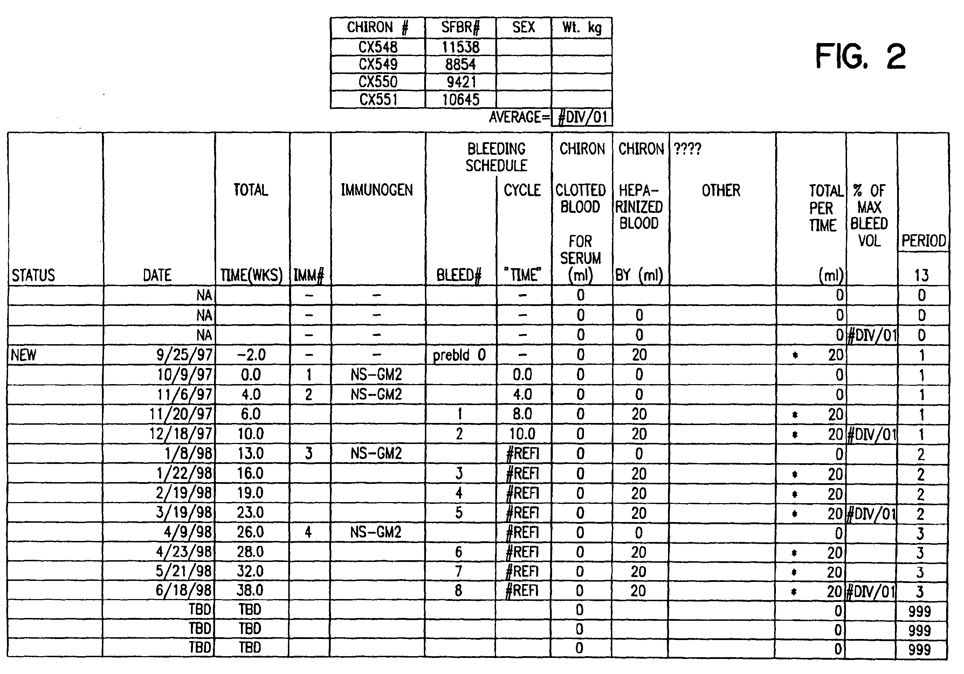

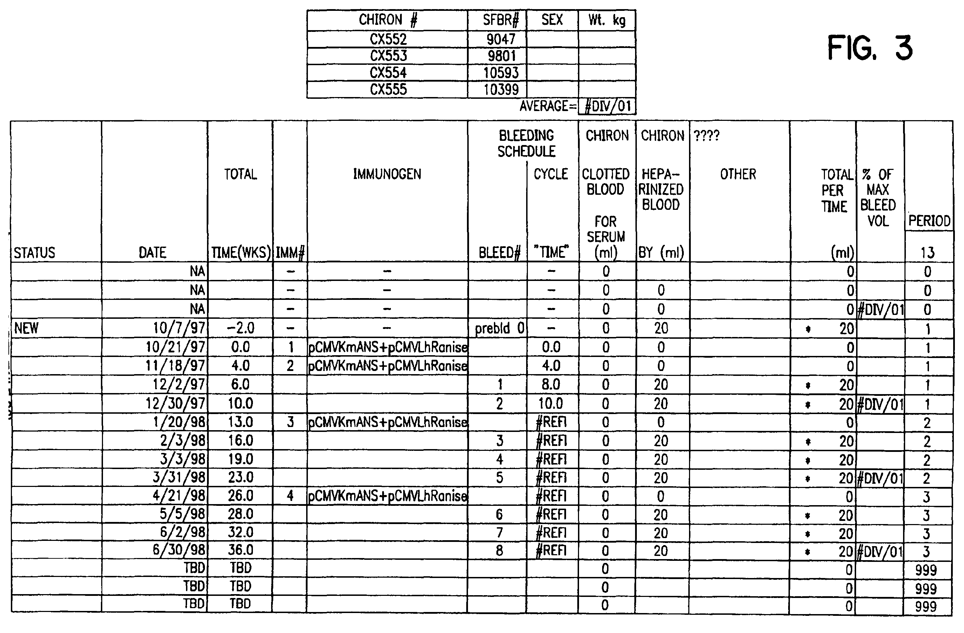

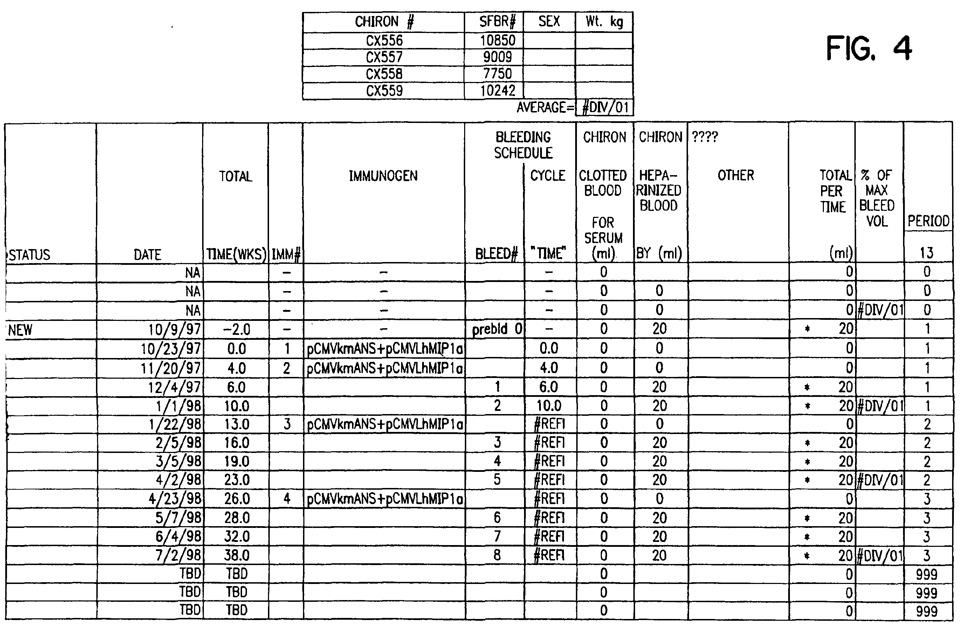

*Immunization and bleeding schedules for four groups of baboons.* Baboons in each of four groups were immunized and bled according to the following schedule. Group 1 (animals CK544, CK545, CK546, and CK547) received inoculations of pCMVKmΔNS (HCV immunogens) and were bled according to the schedule in Figure 1. Group 2 (animals CK548, CK549, CK550, and CK551) received inoculations of NS-GM2 (HCV immunogens and GM-CSF) and were bled according to the schedule in Figure 2. Group 3 (animals CK552, CK553, CK554, and CK555) received inoculations of pCMVKmΔNS and pCMVLhRantes (HCV immunogens and RANTES) according to the schedule in Figure 3. Group 4 (animals CK556, CK557, CK558, and CK559) received inoculations of pCMVKmΔNS and pCMVLhMIP1a (HCV immunogens and MIP-1α) and were bled according to the schedule in Figure 4.

Immunizations were carried out as described in Example 2, above. At each of the times indicated in the bleeding schedules, blood was drawn from the femoral vein while the baboons were under anesthesia (Ketamine®, 10 mg/ml). Blood was treated with heparin. B and T cells were isolated from these blood samples and used in the cytotoxic T lymphocyte assays described below.

*CTL assays.* Autologous B cell lines from each animal were established by transforming B cells with *H. papio.* Separate samples of peripheral blood mononuclear cells were restimulated with immortalized autologous B cells infected with a recombinant vaccinia virus that encodes each of the HCV immunogens (NS3, NS4, NS5a, and NS5b). Two weeks later, CD8⁺ T lymphocytes were purified from the samples using magnetic beads.

The ability of T cells from each animal to lyse its autologous B cell line infected with vaccinia virus encoding the same immunogens used to immunize the animals was tested using a standard ⁵¹Cr-release assay. Ratios of effector (T cells) to target (B cells) of 40:1, 10:1, and 2:1 were tested.

Percent lysis was calculated in each assay. A positive CTL response was noted if at least 10% more lysis occurred with homologous cells (stimulated with a vaccinia virus encoding an HCV immunogen) than with heterologous cells (stimulated with a vaccinia virus encoding an unrelated immunogen) for each of the two highest effector to target cell ratios tested.

Table I shows the number of animals with positive responses in a cytotoxic T lymphocyte assay.

**Table I. Number of animals with CTL responses**

| **Immunogen** | **No. of Animals** |
|---|---|
| pCMVNS3-5 | 0/4 |
| pCMVNS3-5 & MIP-1α | 1/4 |
| pCMVNS3-5 & RANTES | 0/4 |

Table II shows percent lysis of target cells from animal CK556 after restimulation. Homologous cells were stimulated with vaccinia virus encoding HCV polypeptide NS3.

**Table II. Percent lysis of targets after restimulation (animal CK556)**

| | **Effector:Target** | **Homologous**^{**1**} | **Heterologous**^{**2**} |
|---|---|---|---|
| pre-immunization | 40:1 | 2 | 11 |
| pre-immunization | 10:1 | 6 | 10 |
| pre-immunization | 2:1 | 7 | 6 |
| 2 weeks post 3rd immunization | 40:1 | 27 | <1 |
| 2 weeks post 3rd immunization | 10:1 | 17 | <1 |
| 2 weeks post 3rd immunization | 2:1 | 11 | <1 |

| | | | |
|---|---|---|---|
| ¹ stimulated with a vaccinia virus encoding HCV polypeptide NS3. | | | |
| ² stimulated with a vaccinia virus encoding an unrelated immunogen. | | | |

The results reported in Table II demonstrate that co-administration of HCV immunogens and the chemokine MIP-1α resulted in an increased lysis of autologous B cells infected with vaccinia virus encoding HCV polypeptide NS3.

### EXAMPLE 2

### Co-administration of HIV immunogens and BLC increases the titer of anti-p55gag

Balb/c mice received bilateral injections into the anterior tibialis muscle of 10 µg of a p55 plasmid, which encodes HIV gag, either alone or together with a total of 100 µg of a plasmid encoding B lymphocyte chemokine (BLC; *Nature 391,* 799-803, 1998). Fifty µg of BLC-encoding plasmid were injected into each muscle.

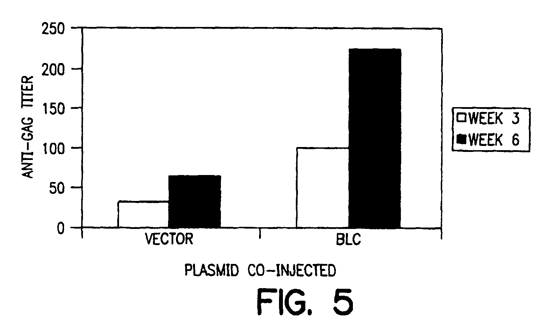

The animals were bled at 3 and 6 weeks after immunization, and anti-p55gag antibody titer was measured by ELISA. Figure 5 shows that anti-gag antibody titer in immunized mice is increased at three weeks after immunization and continues to increase up to at least six weeks.

### NUMBERED REFERENCES

1. Ulmer, J., *et al*. 1993. Heterologous protection against influenze by injection of DNA encoding a viral protein [see comments]. *Science* 259:1745-9.
2. Fynan, E., R. *et al*. 1993. DNA vaccines: protective immunizations by parenteral, mucosal, and gene-gun inoculations. *Proc Natl Acad Sci USA* 90:11478-82.
3. Cox, G., *et al*. 1993. Bovine herpesvirus 1: immune responses in mice and cattle injected with plasmid DNA. *J Virol* 67:5664-7.
4. Sedegah, *M., et al*. 1994. Protection against malaria by immunization with plasmid DNA encoding circumsporozoite protein. *Proc Natl Acad Sci USA* 91:9866-70.
5. Barry, M., *et al.* 1995. Protection against mycoplasma infection using expression-library immunization. *Nature* 377-632-5.
6. Conry, R., *et al.* 1995. A carcinoembryonic antigen polynucleotide vaccine has in vivo antitumor activity. *Gene Ther* 2:59-65.
7. Syrengelas, *A., et al*., 1996. DNA immunization induces protective immunity against B-cell lymphoma. *Nat Med* 2*:1038-1041.*
8. Tascon, R*., et al*. 1996. Vaccination against tuberculosis by DNA injection. *Nat Med* 2:888-92.
9. Yasutomi, Y., *et al.* 1996. Simian immunodeficiency virus-specific cytotoxic T-lymphocyte induction through DNA vaccination of rhesus monkeys. *J Virol* 70:678-81.
10. Letvin, M., *et al*. 1997. Potent protective anti-HIV immune responses generated by bimodal HIV envelope DNA plus protein vaccination. *Proc Natl Acad Sci USA* 94:9378-9383.
11. Xiang, Z., and H. Ertl. 1995. Manipulation of the immune responses to a plasmid-encoded viral antigen by coinoculation with plasmids expressing cytokines. *Immunity* 2:129-35.
12. Conry, R., *et al.* 1996. Selected strategies to augment polynucleotide immunization. *Gene Ther* 3:67-74.
13. Irvine, K., *et al.* 1996. Cytokine enhancement of DNA immunization leads to effective treatment of established pulmonary metastases. *J. Immunol* 156:238-45.
14. Chow, Y., *et al*. 1997. Improvement of hepatitis B virus DNA vaccines by plasmids coexpressing hepatitis B Surface antigen and interleukin-2. *J Virol* 71:169-78.
15. Iwasaki, A., *et al.* 1997. Enhanced CTL responses mediated by plasmid DNA immunogens encoding costimulatory molecules and cytokines. *J Immmunol* 158:4591-601.
16. Kim, J., *et al*. 1997. In vivo engineering of a cellular immune response by coadministration of IL-12 expression vector with a DNA immunogen. *J Immunol* 158:816-26.
17. Okada, E., *et al*. 1997. Intranasal immunization of a DNA vaccine with IL-12- and granulocyte-macrophage colony-stimulating factor (GM-CSF)-expressing plasmids in liposomes induces strong mucosal and cell-mediated immune responses against HIV-1 antigens. *J Immunol* 159:3638-47.
18. Geissler, M., *et al.* 1997. Enhancement of cellular and humoral immune responses to hepatitis C viruws core proteins using DNA-based vaccines augmented with cytokine-expressing plasmids. *J Immunol* 158:1231-7.
19. Larsen, D., *et al*. 1998. Coadministration of DNA encoding interleukin-6 and hemagglutinin confers protection from influenza virus challenge in mice. *J Virol* 72:1704-8.
20. Butcher, E. 1991. Leukocyte-endothelial cell recogtnition: three (or more) steps to specificity and diversity. *Cell* 67:1033-6.
21. Springer, T. 1994. Traffic signals for lymphocyte recirculation and leukocyte emigration: the multistep paradigm. *Cell* 76:301-14.
22. Butcher, E., and L. Picker. 1996. Lymphocyte homing and homeostasis. *Science* 272:60-6.
23. Schall, T., and K. Bacon. 1994. Chemokines, leukocyte trafficking, and inflammation. *Curr Opin Immunol* 6:865-73.
24. Taub, D., *et al.* 1993. Preferential migration of activated CD4+ and CD8+ T cells in response to MIP-1 alpha and MIP-1 beta *Science* 260:355-8.
25. Schall, T., *et al*. 1993. Human macrophage inflammatory protein alpha (MIP-1 alpha) and MIP-1 beta chemokines attract distinct populations of lymphocytes. *J Exp Med* 177:1821-6.
26. Schall, T., *et al.* 1990. Selective attraction of monocytes and T lymphocytes of the memory phenotype by cytokine RANTES. *Nature* 347:669-71.
27. Rot, A., *et al*. 1992. RANTES and macrophage inflammatory protein 1 alpha induce the migration and activation of normal human eosinophil granulocytes. *J Exp Med* 176:1489-95.

## Claims

1. An immunogenic composition comprising:
a DNA immunogen comprising any contiguous sequence of deoxyribonucleotides encoding a polypeptide which is capable of eliciting an immune response; and
a B lymphocyte chemokine (BLC) or a polynucleotide encoding a B lymphocyte chemokine.

2. The immunogenic composition of claim 1 wherein the DNA immunogen comprises a polynucleotide encoding a viral immunogen.

3. The immunogenic composition of claim 2 wherein the polynucleotide encodes a hepatitis C virus non-structural polypeptide.

4. The immunogenic composition of claim 3 wherein the hepatitis C virus non-structural polypeptide is selected from the group consisting of NS3, NS4, NS5a, and NS5b.

5. The immunogenic composition of claim 2 wherein the polynucleotide encodes an HIV polypeptide.

6. The immunogenic composition of claim 5 wherein the HIV polypeptide is a gag polypeptide.

7. The immunogenic composition of claim 1 wherein the DNA immunogen comprises a polynucleotide encoding an immunogen expressed by a tumor.

8. The immunogenic composition of claim 1 further comprising a pharmaceutically acceptable carrier.

9. A product comprising as a combined preparation (i) a B lymphocyte chemokine (BLC) or a first polynucleotide encoding a B lymphocyte chemokine and (ii) a DNA immunogen comprising any contiguous sequence of deoxyribonucleotides encoding a polypeptide which is capable of eliciting an immune response, for simultaneous, separate or sequential use in enhancing an immune response to the DNA immunogen in a mammal.

10. Use of (i) a B lymphocyte chemokine (BLC) or a first polynucleotide encoding a B lymphocyte chemokine and (ii) a DNA immunogen comprising any contiguous sequence of deoxyribonucleotides encoding a polypeptide which is capable of eliciting an immune response, in the manufacture of a medicament for use in enhancing an immune response to the DNA immunogen in a mammal.

11. The use of claim 10 wherein the B lymphocyte chemokine (BLC), or a first polynucleotide encoding the B lymphocyte chemokine, and the DNA immunogen are to be co-administered.

12. The use of claim 10 wherein the B lymphocyte chemokine (BLC), or a first polynucleotide encoding the B lymphocyte chemokine, is to be administered prior to administration of the DNA immunogen.

13. The use of claim 10 wherein the DNA immunogen is to be administered prior to administration of the B lymphocyte chemokine (BLC), or a first polynucleotide encoding the B lymphocyte chemokine.

14. The use of claim 10 wherein a first polynucleotide encoding the B lymphocyte chemokine (BLC) and a second polynucleotide which comprises (a) the first polynucleotide and (b) the DNA immunogen are to be administered.

15. The use of any one of claims 10 to 13 wherein the DNA immunogen comprises a polynucleotide which encodes a hepatitis C virus non-structural polypeptide.

16. The use of claim 15 wherein the hepatitis C virus non-structural polypeptide is selected from the group consisting of NS3, NS4, NS5a, and NS5b.

17. The use of any one of claims 10 to 13 wherein the polynucleotide encodes an HIV polypeptide.

18. The use of claim 17 wherein the HIV polypeptide is a gag polypeptide.

19. The use of any one of claims 10 to 18 wherein the mammal is a human.

20. The use of any one of claims 10 to 19 wherein the immune response is an antibody response.

21. The use of any one of claims 10 to 19 wherein the immune response is a cytotoxic T lymphocyte response.

## Patentansprüche

1. Immunogene Zusammensetzung, umfassend:
ein DNA-Immunogen, umfassend eine zusammenhängende Sequenz von Desoxyribonucleotiden codierend ein Polypeptid, das in der Lage ist, eine Immunantwort auszulösen; und
ein B-Lymphozytenchemokin (BLC) oder ein Polynucleotid, das ein B-Lymphozytenchemokin codiert.

2. Immunogene Zusammensetzung nach Anspruch 1, wobei das DNA-Immunogen ein Polynucleotid umfasst, das ein virales Immunogen codiert.

3. Immunogene Zusammensetzung nach Anspruch 2, wobei das Polynucleotid ein nicht-strukturelles Polypeptid des Hepatitis C Virus codiert.

4. Immunogene Zusammensetzung nach Anspruch 3, wobei das nicht-strukturelle Polypeptid des Hepatitis C Virus aus der Gruppe bestehend aus NS3, NS4, NS5a und NS5b ausgewählt wird.

5. Immunogene Zusammensetzung nach Anspruch 2, wobei das Polynucleotid ein HIV-Polypeptid codiert.

6. Immunogene Zusammensetzung nach Anspruch 5, wobei das HIV-Polypeptid ein GAG-Polypeptid ist.

7. Immunogene Zusammensetzung nach Anspruch 1, wobei das DNA-Immunogen ein Polynucleotid umfasst, das ein von einem Tumor exprimiertes Immunogen codiert.

8. Immunogene Zusammensetzung nach Anspruch 1, weiterhin umfassend einen pharmazeutisch-verträglichen Träger.

9. Produkt, umfassend als kombinierte Präparation (i) ein B-Lymphozytenchemokin (BLC) oder ein erstes Polynucleotid, das ein B-Lymphozytenchemokin codiert, und (ii) ein DNA-Immunogen, umfassend eine zusammenhängende Sequenz von Desoxyribonucleotiden codierend ein Polypeptid, das in der Lage ist, eine Immunantwort auszulösen, zur gleichzeitigen, getrennten oder aufeinanderfolgenden Verwendung zur Verstärkung einer Immunantwort in einem Säuger gegen das DNAlmmunogen.

10. Verwendung von (i) einem B-Lymphozytenchemokin (BLC) oder einem ersten Polynucleotid, das ein B-Lymphozytenchemokin codiert, und (ii) einem DNA-Immunogen, umfassend eine zusammenhängende Sequenz von Desoxyribonucleotiden codierend ein Polypeptid, das in der Lage ist, eine Immunantwort auszulösen, zur Herstellung eines Medikaments zur Verwendung bei der Verstärkung einer Immunantwort in einem Säuger gegen das DNA-Immunogen.

11. Verwendung nach Anspruch 10, wobei das B-Lymphozytenchemokin (BLC) oder ein erstes Polynucleotid, das das B-Lymphozytenchemokin codiert, und das DNA-Immunogen gleichzeitig zu verabreichen sind.

12. Verwendung nach Anspruch 10, wobei das B-Lymphozytenchemokin (BLC), oder ein erstes Polynucleotid, das das B-Lymphozytenchemokin codiert, vor der Verabreichung des DNA-Immunogens zu verabreichen ist.

13. Verwendung nach Anspruch 10, wobei das DNA-Immunogen vor Verabreichung des B-Lymphozytenchemokins (BLC) oder eines ersten Polynucleotids, das das B-Lymphozytenchemokin codiert, zu verabreichen ist.

14. Verwendung nach Anspruch 10, wobei ein erstes Polynucleotid, das das B-Lymphozytenchemokin (BLC) codiert, und ein zweites Polynucleotid, das (a) das erste Polynucleotid und (b) das DNA-Immunogen umfasst, zu verabreichen sind.

15. Verwendung nach einem der Ansprüche 10 bis 13, wobei das DNA-Immunogen ein Polynucleotid umfasst, das ein nicht-strukturelles Polypeptid des Hepatitis C Virus codiert.

16. Verwendung nach Anspruch 15, wobei das nicht-strukturelle Polypeptid des Hepatitis C Virus aus der Gruppe bestehend aus NS3, NS4, NS5a und NS5b ausgewählt wird.

17. Verwendung nach einem der Ansprüche 10 bis 13, wobei das Polynucleotid ein HIV-Polypeptid codiert.

18. Verwendung nach Anspruch 17, wobei das HIV-Polypeptid ein GAG-Polypeptid ist.

19. Verwendung nach einem der Ansprüche 10 bis 18, wobei der Säuger ein Mensch ist.

20. Verwendung nach einem der Ansprüche 10 bis 19, wobei die Immunantwort eine Antikörperantwort ist.

21. Verwendung nach einem der Ansprüche 10 bis 19, wobei die Immunantwort eine cytotoxische T-Lymphozytenantwort ist.

## Revendications

1. Composition immunogène comprenant :
un ADN immunogène comprenant n'importe quelle séquence contiguë de désoxyribonucléotides codant pour un polypeptide qui est capable d'induire une réponse immunitaire ; et
une chimiokine pour lymphocytes B (BLC) ou un polynucléotide codant pour une chimiokine pour lymphocytes B.

2. Composition immunogène selon la revendication 1, dans laquelle l'ADN immunogène comprend un polynucléotide codant pour un immunogène viral.

3. Composition immunogène selon la revendication 2, dans laquelle le polynucléotide code pour un polypeptide non structural du virus de l'hépatite C.

4. Composition immunogène selon la revendication 3, dans laquelle le polypeptide non structural du virus de l'hépatite C est choisi dans le groupe constitué par NS3, NS4, NS5a et NS5b.

5. Composition immunogène selon la revendication 2, dans laquelle le polynucléotide code pour un polypeptide du VIH.

6. Composition immunogène selon la revendication 5, dans laquelle le polypeptide du VIH est un polypeptide gag.

7. Composition immunogène selon la revendication 1, dans laquelle l'ADN immunogène comprend un polynucléotide codant pour un immunogène exprimé par une tumeur.

8. Composition immunogène selon la revendication 1, comprenant en outre un véhicule pharmaceutiquement acceptable.

9. Produit comprenant en tant que préparation combinée (i) une chimiokine pour lymphocytes B (BLC) ou un premier polynucléotide codant pour une chimiokine pour lymphocytes B et (ii) un ADN immunogène comprenant n'importe quelle séquence contiguë de désoxyribonucléotides codant pour un polypeptide qui est capable d'induire une réponse immunitaire pour une utilisation simultanée, distincte ou séquentielle pour l'amélioration d'une réponse immunitaire à l'ADN immunogène chez un mammifère.

10. Utilisation (i) d'une chimiokine pour lymphocytes B (BLC) ou d'un premier polynucléotide codant pour une chimiokine pour lymphocytes B et (ii) d'un ADN immunogène comprenant n'importe quelle séquence contiguë de désoxyribonucléotides codant pour un polypeptide qui est capable d'induire une réponse immunitaire à l'ADN immunogène chez un mammifère.

11. Utilisation selon la revendication 10, dans laquelle la chimiokine pour lymphocytes B (BLC) ou un premier polynucléotide codant pour la chimiokine pour lymphocytes B et l'ADN immunogène doivent être co-administrés.

12. Utilisation selon la revendication 10, dans laquelle la chimiokine pour lymphocytes B (BLC) ou un premier polynucléotide codant pour la chimiokine pour lymphocytes B doivent être administrés avant d'administrer l'ADN immunogène.

13. Utilisation selon la revendication 10, dans laquelle l'ADN immunogène doit être administré avant d'administrer la chimiokine pour lymphocytes B (BLC) ou un premier polynucléotide codant pour la chimiokine pour lymphocytes B.

14. Utilisation selon la revendication 10, dans laquelle un premier polynucléotide codant pour la chimiokine pour lymphocytes B (BLC) et un second polynucléotide qui comprend (a) le premier polynucléotide et (b) l'immunogène doivent être administrés.

15. Utilisation selon l'une quelconque des revendications 10 à 13, dans laquelle l'ADN immunogène comprend un polynucléotide qui code pour un polypeptide non structural du virus de l'hépatite C.

16. Utilisation selon la revendication 15, dans laquelle le polypeptide non structural du virus de l'hépatite C est choisi dans le groupe constitué par NS3, NS4, NS5a et NS5b.

17. Utilisation selon l'une quelconque des revendications 10 à 13, dans laquelle le polynucléotide code pour un polypeptide du VIH.

18. Utilisation selon la revendication 17, dans laquelle le polypeptide du VIH est un polypeptide gag.

19. Utilisation selon l'une quelconque des revendications 10 à 18, dans lequel le mammifère est un être humain.

20. Utilisation selon l'une quelconque des revendications 10 à 19, dans laquelle la réponse immunitaire est une réponse anticorps.

21. Utilisation selon l'une quelconque des revendications 10 à 19, dans laquelle la réponse immunitaire est une réponse des lymphocytes T cytotoxiques.
